# EUROPEAN PATENT SPECIFICATION

(11) **EP 2 120 803 B1**
(45) Date of publication and mention of the grant of the patent: **06.06.2018**
(21) Application number: 07869825.5
(22) Date of filing: 21.12.2007
(51) Int. Cl.: A61F 2/95

(54) **STENT DELIVERY SYSTEM**
SYSTEM ZUM ANBRINGEN EINES STENTS
SYSTÈME DE DISTRIBUTION DE TUTEUR BILIAIRE

(30) Priority: 05.01.2007 US 620437
(43) Date of publication of application: 25.11.2009
(73) Proprietor: Boston Scientific Limited, Hamilton HM11 (BM)
(72) Inventor: LI, Changqing, Bloomington, IN 47404 (US); WELDON, James, M., Newton, MA 02459 (US); BENNING, Christopher, A., Lowell, MA 01852 (US); JORDAN, Gary, A., Litchfield, NH 03052 (US)
(74) Representative: Vossius & Partner Patentanwälte Rechtsanwälte mbB
(86) International application number: PCT/US2007/088695
(87) International publication number: WO 2008/085714

(56) References cited:
- WO-A-94/11038
- WO-A-02/074378
- WO-A-2005/053574

## Description

### Technical Field

The present disclosure pertains generally to medical devices and more particularly to systems, assemblies and apparatus for use in delivering a stent within a body cavity.

### Background

In medical procedures, stents have been utilized in treating an obstructed body cavity and/or maintaining the patency of a body cavity. Stents may be used in a variety of medical applications. Some suitable examples of stents include, without limitation, ureteral, urethral, pancreatic, vascular, neurovascular, and gastrointestinal stents, for example. Some stents, used in renal procedures, may be used to bypass and/or open an obstructed lumen, such as a duct of the biliary tree or a ureter, and may often be configured for long-term positioning within the lumen. Such stents, interchangeably known as drainage catheters, may be useful in reestablishing patent flow through a renal passageway. Other stents, used in vascular procedures, may be used to open an obstruction, such as a stenosis of a vessel.

Stents or drainage catheters have been found to be highly useful. However, the procedures and/or apparatus involved in positioning a stent or drainage catheter within a body cavity often involve a significant duration of time and/or may require one or more device exchanges. Therefore, a need remains for an improved system, assembly and/or apparatus for delivering a stent or drainage catheter within a body cavity.

WO94/11038 discloses an intravascular catheter comprising a multi-lumen proximal segment.

### Summary

The present invention is defined by the features of the claims.

The disclosure is directed to systems, assemblies, apparatus, and methods for placing a stent within a body cavity.

Accordingly, one illustrative embodiment is a catheter shaft including a distal segment having a proximal end, a distal end, an internal stepped portion proximate the proximal end, and a lumen extending therethrough, and a multi-lumen proximal segment having a proximal end, a distal end, a first lumen and a second lumen. The distal segment is secured to the proximal segment such that each of the first and second lumens of the proximal segment is in association with the lumen of the distal segment. The distal end of the multi-lumen proximal segment is formed at an oblique angle defining a ramp. Further, the stepped portion has an increased inner diameter relative to a more distant portion of the distal segment.

Another illustrative embodiment is a push catheter for placement of a biliary stent. The push catheter includes a hub assembly and an elongate shaft extending from the hub assembly. The elongate shaft includes a distal segment having a lumen extending therethrough, and a proximal segment having a first lumen and a second lumen. The distal segment is secured to the proximal segment such that each of the first and second lumens of the proximal segment is in association with the lumen of the distal segment. The distal end of the proximal segment is formed at an oblique angle defining a ramp. The elongate shaft also includes a side port extending through a side wall of the proximal segment, providing access to the first lumen.

Yet another illustrative embodiment is a biliary stent delivery system including a guide catheter including a tubular member and a pull wire, a stent positioned about the tubular member of the guide catheter, and a push catheter. The push catheter includes a distal segment having a lumen extending therethrough, and a proximal segment having a first lumen and a second lumen. The distal segment is secured to the proximal segment such that each of the first and second lumens of the proximal segment is in association with the lumen of the distal segment. The pull wire of the guide catheter is positioned within the second lumen of the proximal segment of the push catheter. The distal end of the proximal segment may be formed at an oblique angle relative to the longitudinal axis of the proximal segment, defining a ramp.

A further illustrative embodiment is a tool for urging an elongate shaft of a catheter into a curved orientation. The tool comprises a body portion, a securement portion and a biasing portion. The securement portion includes a first leg extending from the body portion and a second leg extending from the body portion. The second leg is spaced from the first leg such that the first and second legs are configured to retain the elongate shaft therebetween. The biasing portion includes a third leg extending from the body portion, wherein the third leg is misaligned from the first and second legs such that placement of the elongate shaft adjacent the third leg biases the elongate shaft into a curved orientation.

The above summary of some example embodiments is not intended to describe each disclosed embodiment or every implementation of the invention. Although some illustrative embodiments are directed to biliary stent delivery systems, other suitable embodiments include, but are not necessarily limited to, ureteral, urethral, pancreatic, vascular, neurovascular, and gastrointestinal stents, and the like.

### Brief Description of the Drawings

The invention may be more completely understood in consideration of the following detailed description of various embodiments in connection with the accompanying drawings, in which:
FIG. 1 is a plan view of an exemplary stent delivery system;
FIG. 2 is a plan view of an exemplary delivery catheter;
FIG. 3 is a cross-sectional view of the exemplary delivery catheter of FIG. 2;
FIG. 4 is a plan view of an exemplary push catheter;
FIG. 5 is an enlarged cross-sectional view of a portion of the exemplary push catheter of FIG. 4;
FIG. 5A is a cross-sectional view taken along line 5A-5A of FIG. 5;
FIG. 5B is a cross-sectional view taken along line 5B-5B of FIG. 5;
FIG. 6 is an alternative enlarged cross-sectional view of a portion of the exemplary push catheter of FIG. 4;
FIG. 6A is a cross-sectional view taken along line 6A-6A of FIG. 6;
FIG. 6B is a cross-sectional view taken along line 6B-6B of FIG. 6;
FIG. 7 is an alternative enlarged cross-sectional view of a portion of an exemplary push catheter;
FIG. 7A is a cross-sectional view taken along line 7A-7A of FIG. 7;
FIG. 7B is a cross-sectional view taken along line 7B-7B of FIG. 7;
FIG. 8 is an alternative enlarged cross-sectional view of a portion of an exemplary push catheter;
FIG. 8A is a cross-sectional view taken along line 8A-8A of FIG. 8;
FIG. 8B is a cross-sectional view taken along line 8B-8B of FIG. 8;
FIGS. 9A-9D illustrate a progression of directing a guidewire through a lumen and out a side port of a catheter shaft; and
FIG. 10 is an exemplary tool for providing a curvature to a catheter shaft.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the invention.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the invention. The illustrative embodiments depicted are intended only as exemplary. Selected features of any illustrative embodiment may be incorporated into an additional embodiment unless clearly stated to the contrary.

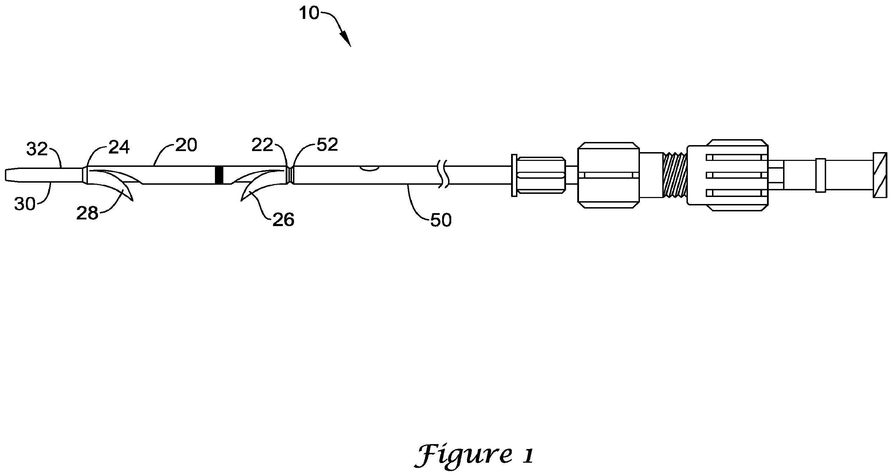

Referring now to FIG. 1, a system for delivering a stent is generally shown. The stent delivery system 10, which may be assembled prior to insertion into the body of a patient, either by the manufacturer or by the physician, includes a stent 20, a delivery catheter 30, and a push catheter 50. The stent 20, which includes a proximal end 22 and a distal end 24, may be disposed on the body portion 32 of the delivery catheter 30. The push catheter 50 may extend over the delivery catheter 30 such that the distal end 52 of the push catheter 50 abuts the proximal end 22 of the stent 20.

The stent 20 may be formed of any suitable material, such as a metallic material or a polymeric material. Some suitable metallic materials include, but are not necessarily limited to, stainless steel, tungsten, nickel-titanium alloys such as those possessing shape memory properties commonly referred to as nitinol, nickel-chromium alloys, nickel-chromium-iron alloys, or other suitable metals, or combinations or alloys thereof. Some suitable polymeric materials include, but are not necessarily limited to, polyamide, polyether block amide, polyethylene, polypropylene, polyvinylchloride, polyurethane, polytetrafluoroethylene, and copolymers, blends, mixtures or combinations thereof.

The stent 20 may include a retention structure to prevent migration of the stent 20 within a body cavity. For example, in some embodiments the stent 20 may include a first barb 26 and/or a second barb 28 which may assist in maintaining the stent 20 in a desired position within a body cavity. In some embodiments, the barbs 26, 28 may be arranged in an opposing orientation such that the barbs 26, 28 may prevent the stent 20 from migrating in either axial direction. In other embodiments, the stent 20 may include additional and/or alternative retention means which may prevent displacement of the stent 20 once positioned within a body cavity.

The stent 20 may be selectively connected to the push catheter 50 using a retention device (not shown), such as a suture. For example, U.S. Pat. No. 6,264,624 to Desmond, III et al. and U.S. Pat. No. 6,562,024 to Alvarez de Toledo et al., the disclosures of which are incorporated herein by reference, disclose the use of a suture as one such retention device. One of skill in the art would recognize other retention devices may be used to selectively removably connect the stent 20 to the push catheter 50.

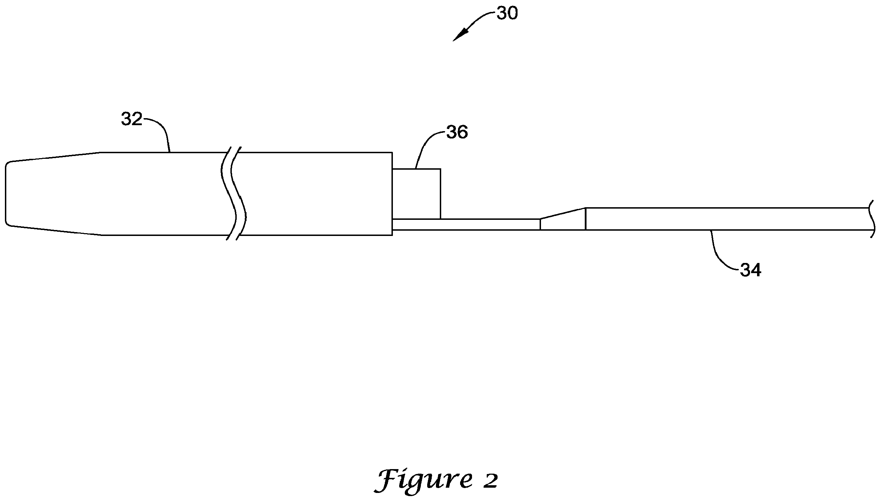

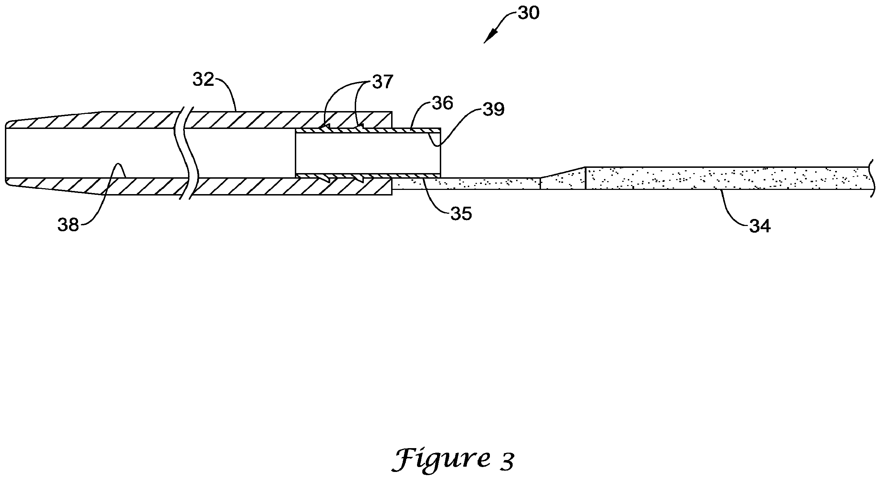

The delivery catheter 30 is further described in FIGS. 2 and 3. The delivery catheter 30 may include a body portion 32 and a pull wire 34. In some embodiments, the body portion 32 may be a tubular member having a lumen 38 extending therethrough, and in some embodiments, the pull wire 34 may be a monofilament wire or a multi-filament wire, such as a braided wire. The body portion 32 and the pull wire 34 may each be formed of any suitable material, such as a polymeric material or a metallic material including, but not necessarily limited to, those materials listed elsewhere herein. In some embodiments, the body portion 32 may be formed of a material dissimilar to that of the pull wire 34. For instance, in some embodiments, the body portion 32 may be formed of a polymeric material while the pull wire 34 may be formed of a metallic material.

In instances wherein the body portion 32 comprises a polymeric material and the pull wire 34 comprises a metallic material, a coupling 36 may be used to couple the body portion 32 to the pull wire 34. In some embodiments, the coupling 36 may be a metallic member compatible with the metallic material of the pull wire 34. Thus, the pull wire 34 may be attached to the coupling 36 at the attachment location 35. For instance, the pull wire 34 may be attached to the coupling 36 by welding, brazing, soldering, or the like. In other embodiments, the coupling 36 and the pull wire 34 may be a unitary member such that the coupling 36 may be integral with the pull wire 34. As shown in FIG. 3, the coupling 36 may be a tubular member having a lumen 39 extending therethrough. In some embodiments the lumen 39 of the coupling 36 may be co-axial with the lumen 38 of the body portion 32, providing a passageway therethrough for the placement of another medical device, such as a guidewire.

The coupling 36 may include one or more barbs 37, such as annular ridges, extending around the circumference of the coupling 36. For instance, the coupling 36 shown in FIG. 3 includes two barbs 37 positioned at two longitudinally spaced apart locations. The barbs 37 may extend around the entire circumference of the coupling 36, or the barbs 37 may extend only partially around the coupling 36 such as at radially spaced apart locations. The barbs 37 may be used to secure the body portion 32 of the delivery catheter 30 to the coupling 36, and thus the pull wire 34. For instance, the proximal portion of the body portion 32 may be inserted over the coupling 36 and urged proximally such that a portion of the body portion 32 extends over and past the barbs 37. The radial extents of the barbs 37 may be greater than the inside diameter of the body portion 32, such that the barbs 37 create an interference fit with the body portion 32. The orientation of the barbs 37 deters subsequent distal movement of the body portion 32 relative to the coupling 36, and thus inhibits detachment of the body portion 32 from the pull wire 34. In some embodiments, a length of heat shrink tubing (not shown) may be placed about the body portion 32 overlaying the coupling 36 and then heat shrunk in place in order to further retain the body portion 32 to the coupling 36.

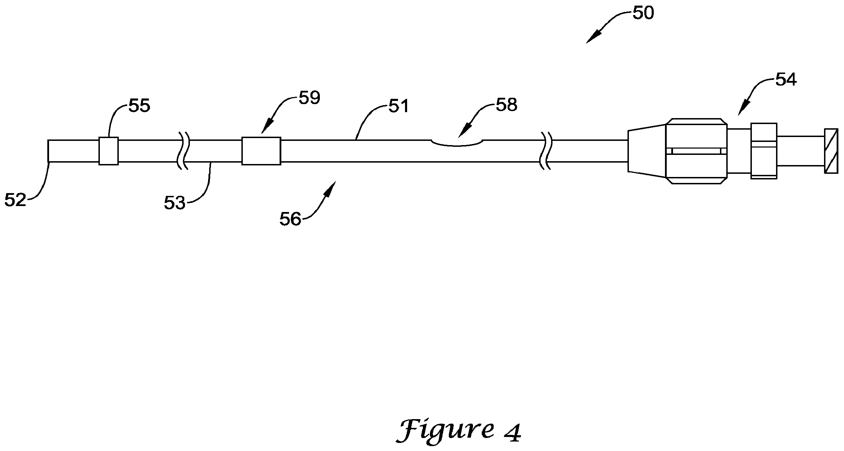

The push catheter 50 is further described in FIG. 4. The push catheter 50 may include a hub assembly 54 and an elongate shaft 56 extending distally therefrom. In some embodiments, the elongate shaft 56 may include a radiopaque marker 55 proximate the distal end 52 of the elongate shaft 56, or at another location along the elongate shaft 56. The radiopaque marker 55, if present, may aid a physician in positioning the stent 20 (shown in FIG. 1) during a medical procedure.

The elongate shaft 56 may include multiple sections such as a proximal section 51 and a distal section 53. In some embodiments, the proximal section 51 may be a tubular member extrusion and the distal section 53 may be a tubular member extrusion distinct from the proximal section 51. In such embodiments, the proximal section 51 may be attached to the distal section 53 at a joint 59 during a post-extrusion process. The proximal section 51 may be attached to the distal section 53 in any suitable fashion, such as fusion bonding (e.g., laser bonding), adhesive bonding, RF welding, compression fit, heat shrink connection, or the like.

In some embodiments, the proximal section 51 may be formed of a polymeric material different from the polymeric material of the distal section 53. In other embodiments, the proximal section 51 and the distal section 53 may be formed of a similar polymeric material. In some embodiments, the proximal section 51 may have a durometer hardness different from the durometer hardness of the distal section 53. For instance, the durometer hardness of the proximal section 51 may be greater than or less than the durometer hardness of the distal section 53. In some embodiments, the durometer hardness of the proximal section 51 may be in the range of about 60D to about 90D, in the range of about 70D to about 80D, or in the range of about 70D to about 75D on the Shore hardness scale. In some embodiments, the durometer hardness of the distal section 53 may be in the range of about 40D to about 80D, in the range of about 50D to about 70D, or in the range of about 60D to about 70D on the Shore hardness scale. Although some suitable hardness values are disclosed, one of skill in the art, incited by the present disclosure, would understand that desired values may deviate from those expressly disclosed.

In some embodiments, it is possible to have more than two sections of differential stiffness. For instance, in some embodiments, the elongate shaft 56 may have three, four, or more sections or regions of differential stiffness. For example, in some embodiments, the elongate shaft 56 may have a first section with a first durometer hardness, a second section with a second durometer hardness greater than the first durometer hardness, and a third section with a third durometer hardness greater than both the first durometer hardness and the second durometer hardness. Other embodiments may include additional sections, such as a fourth section with a fourth durometer hardness greater than the first, second and third durometer hardness.

In other embodiments, other means may be utilized in order to provide the elongate shaft 56 of the push catheter 50 with multiple sections or regions of differential stiffness. For example, one could switch from a first polymer to a second polymer having different stiffness properties during extrusion of one or more sections of the elongate shaft 56. Alternatively, the elongate shaft 56 may be formed of multiple layers of material along its length. Thus, fewer and/or thinner layers of material may be located throughout regions which are desired to be softer than adjacent regions of the elongate shaft 56, for example. In other embodiments, one or more portions of the elongate shaft 56 may be reinforced with one or more reinforcement members, such as braids, coils, strips of coextruded material, heatshrink sleeves, elongate fibers, ribbing, etc. In still other embodiments, the sidewall of one or more select portions of the elongate shaft 56 may have a reduced thickness compared to an adjacent portion of the elongate shaft 56. Thus, the one or more regions of reduced thickness may be less stiff (e.g., have greater flexibility) than adjacent, thicker regions of the elongate shaft 56. Still other design choices may provide the elongate shaft 56 with multiple regions of differential flexibility or other desired characteristics.

The elongate shaft 56 may include a side port 58 providing access to the interior of the elongate shaft 56. For instance, the side port 58 may provide a guidewire port for placement of a guidewire within a lumen of the elongate shaft 56. In some embodiments, the side port 58 may be a skived portion of the elongate shaft 56, for example. In the illustrative embodiment, the side port 58 is formed in the proximal section 51 of the elongate shaft 56. However, in other embodiments, the side port 58 may be formed in the distal section 53, if desired.

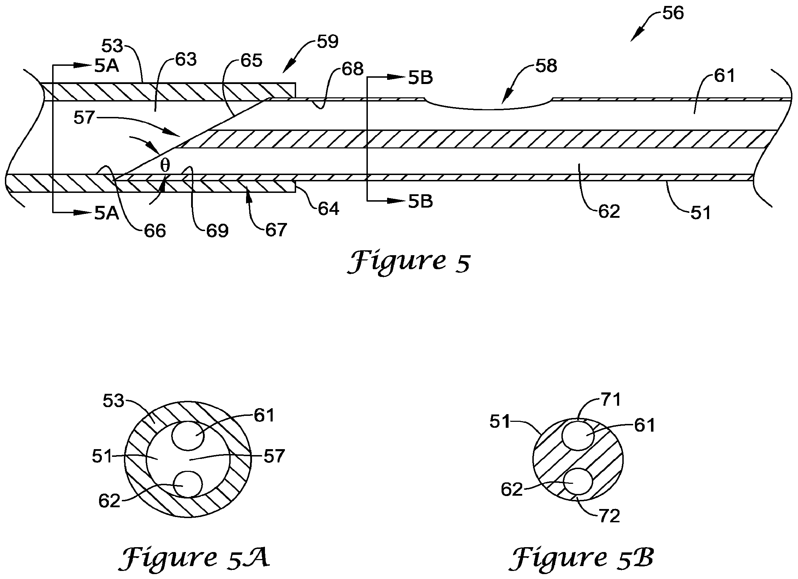

FIG. 5 is an enlarged cross-sectional view of a portion of the elongate shaft 56 of the push catheter 50 (shown in FIG. 4) including the joint 59 between the distal section 53 and the proximal section 51 and the side port 58. In some embodiments, such as that illustrated in FIG. 5, the distal section 53 may be a single-lumen tubular member and the proximal section 51 may be a multi-lumen tubular member having two or more lumens. For instance, as shown in FIG. 5, the distal section 53 includes a lumen 63 for receiving a portion of the proximal section 51. Furthermore, as shown in FIG. 5B, the proximal section 51 may be a dual-lumen tubular member having a first lumen 61 and a second lumen 62. Each of the first lumen 61 and the second lumen 62 may be in association (e.g., in fluid communication) with the lumen 63 of the distal section 53. The side port 58 may provide access to the first lumen 61 of the proximal section 51.

As shown in FIG. 5A, the proximal section 51 may be positioned within the lumen 63 of the distal section 53 such that the perimeter (e.g., circumference) of the first lumen 61 is tangent to the perimeter (e.g., circumference) of the lumen 63 of the distal section 53. Additionally or alternatively, the proximal section 51 may be positioned within the lumen 63 of the distal section 53 such that the perimeter (e.g., circumference) of the second lumen 62 is tangent to the perimeter (e.g., circumference) of the lumen 63 of the distal section 53. As used herein, the term "tangent" is intended to mean a line, curve, or surface meeting another line, curve, or surface at a common point and/or sharing a common tangent line or tangent plane at that point. Thus, as shown in FIG. 5A, the curvature of the circumference of the inner surface of the lumen 63 meets the curvature of the circumference of the inner surface of the first lumen 61 and/or the curvature of the circumference of the inner surface of the second lumen 62, and the lumen 63 shares a common tangent line or tangent plane at that point with the first lumen 61 and/or the second lumen 62. Such an orientation may be found to facilitate advancing an elongate shaft, such as a guidewire, from the lumen 63 of the distal section 53 into the first lumen 61 and/or the second lumen 62 of the proximal section 51.

In some embodiments, the first lumen 61, which may be considered a guidewire lumen, may extend to the proximal end of the elongate shaft 56. In such embodiments, the push catheter 50 may be optionally used as either an over-the-wire type catheter (where a guidewire is positioned within the first lumen 61 and extends throughout the length of the elongate shaft 56), or as a rapid-exchange type catheter (where the guidewire is positioned within the first lumen 61 through a distal portion of the push catheter 50, exits the push catheter 50 at the side port 58, and extends exterior to the push catheter 50 throughout a portion of the push catheter 50 proximal of the side port 58). Thus, at the discretion of the physician or other operator, the push catheter 50 may be operated as either a rapid-exchange catheter or as an over-the-wire catheter.

In some embodiments, such as shown in FIG. 5, the distal end 65 of the proximal section 51 may be formed at an oblique angle θ to the longitudinal axis of the proximal section 51. For instance, the oblique angle θ may be in the range of about 10 degrees to about 60 degrees, in the range of about 20 degrees to about 50 degrees, or in the range of about 30 degrees to about 45 degrees in some embodiments. Although some suitable values are disclosed, one of skill in the art, incited by the present disclosure, would understand that desired values may deviate from those expressly disclosed.

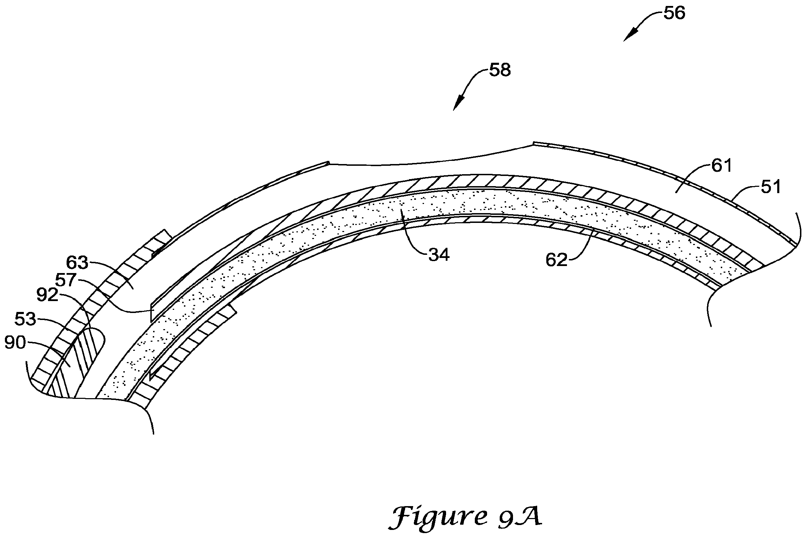

The oblique angle θ of the distal end 65 of the proximal section 51 forms a ramp 57 which may be used to facilitate directing a guidewire from the single lumen 63 of the distal section 53 to the first lumen 61 of the proximal section 51. The ramp 57 may also be viewed through the lumen 63 of the distal section 53 in the cross-section shown in FIG. 5A. Further discussion of the functionality of the ramp 57 will be described while discussing FIGS. 9A-9D.

In some embodiments, such as the illustrative embodiment of FIG. 5, the side port 58 may be spaced apart from the joint 59 between the proximal section 51 and the distal section 53, and thus the ramp 57. For instance, the side port 58 may be located a distance proximal of the ramp 57. In some embodiments, the side port 58 may be located about 2 to about 10 centimeters, about 4 to about 6 centimeters, or about 5 centimeters proximal of the ramp 57. Although some suitable dimensions are disclosed, one of skill in the art, incited by the present disclosure, would understand that desired dimensions may deviate from those expressly disclosed. Placing the side port 58 at a location proximal of the ramp 57 may reduce the potential of kinking of the elongate shaft 56.

As shown in the embodiment illustrated in FIG. 5, the distal end 65 of the proximal section 51 is be positioned within the lumen 63 of the distal section 53 and the outer surface of a distal portion of the proximal section 51 secured to the distal section 53. Thus, the outer diameter of the proximal section 51 is be sized to fit within the lumen 63 of the distal section 53. In some embodiments, the lumen 63 of the distal section 53 may have a stepped portion 67 proximate the proximal end 64 of the distal section 53 for receiving the distal portion of the proximal section 51. The internal stepped portion 67 may be bored out, or otherwise have an increased inner diameter relative to a more distal portion of the distal section 53. Thus, in such embodiments, the outer diameter of the proximal section 51 may be similar to the inner diameter of the internal stepped portion 67, and thus may be greater than the inner diameter of a more distal portion of the distal section 53.

In such an embodiment, the radial distance between the inner wall 66 of the lumen 63 of the distal section 53 and the inner wall 68 of the first lumen 61 of the proximal section 51 and/or the inner wall 69 of the second lumen 62 of the proximal section 51 may be reduced and/or eliminated. Thus, the edge of the distal end 65 of the proximal section 51, which could impede the proximal advancement of a guidewire through the lumen 63 into the first lumen 61 of the proximal section 51, may be reduced or eliminated.

As shown in FIG. 5B, the outer wall 71 of the first lumen 61 may be thinner than the outer wall 72 of the second lumen 62. In some embodiments, the outer wall 71 of the first lumen 61 may be about 0.075 millimeters to about 0.125 millimeters (0.003 inches to about 0.005 inches) in thickness. Although some suitable dimensions are disclosed, one of skill in the art, incited by the present disclosure, would understand that desired dimensions may deviate from those expressly disclosed. As discussed later herein, the offset first lumen 61 and/or the relatively thin outer wall 71 may facilitate advancing a guidewire through the first lumen 61 and/or through the side port 58.

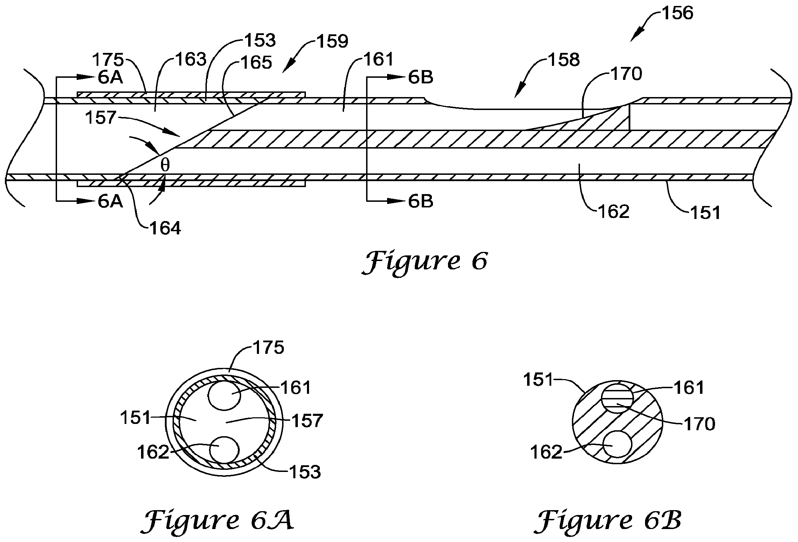

An alternative embodiment of a portion of an elongate shaft 156 including a joint 159 between a distal section 153 and a proximal section 151 and a side port 158 is shown in FIG. 6. In many respects, the portion of the elongate shaft 156 may be similar to the portion of the elongate shaft 56 of the push catheter 50 as shown in FIG. 5. For example, the proximal section 151 may have different flexibility characteristics from the distal section 153. Thus, for the sake of repetitiveness, similarities of the portion of the elongate shaft 156 shown in FIG. 6 with those of the portion of the elongate shaft 56 shown in FIG. 5 will not be repeated.

In FIG. 6, a ramp 170 is illustratively positioned within the first lumen 161 proximate the side port 158. The ramp 170 may be placed within the first lumen 161 in order to direct a guidewire extending through the first lumen 161 exterior of the elongate shaft 156 through the side port 158. The ramp 170 may be a separate member positioned and secured within the first lumen 161, or the ramp 170 may be integrally formed in the proximal section 151 of the elongate shaft 156, such as during formation of the side port 158.

The proximal section 151 may be attached to the distal section 153, such as at joint 159, such that each of the first lumen 161 and the second lumen 162 of the proximal section 151 are in association (e.g., in fluid communication) with the lumen 163 of the distal section 153. Additionally, the distal end 165 of the proximal section 151 may be formed at an oblique angle θ to the longitudinal axis of the proximal section 151. For instance, the oblique angle θ may be in the range of about 10 degrees to about 60 degrees, in the range of about 20 degrees to about 50 degrees, or in the range of about 30 degrees to about 45 degrees in some embodiments. Although some suitable values are disclosed, one of skill in the art, incited by the present disclosure, would understand that desired values may deviate from those expressly disclosed.

The oblique angle θ of the distal end 165 of the proximal section 151 forms a ramp 157 which may be used to facilitate directing a guidewire from the single lumen 163 of the distal section 153 to the first lumen 161 of the proximal section 151. The ramp 157 may also be viewed through the lumen 163 of the distal section 153 in the cross-section shown in FIG. 6A.

In the embodiment illustrated in FIG. 6, the distal end 165 of the proximal section 151 is shown in abutment with and secured to the proximal end 164 of the distal section 153. Thus, the outer diameter of the proximal section 151 may be substantially equivalent to the outer diameter of the distal section 153. Optionally, as shown in FIG. 6, in some embodiments, a connector 175, such as a length of heat shrink tubing, may be placed over the joint 159 between the proximal section 151 and the distal section 153 in order to further secure the proximal section 151 with the distal section 153.

In some embodiments, such as the illustrative embodiment of FIG. 6, the side port 158 may be spaced apart from the joint 159 between the proximal section 151 and the distal section 153, and thus the ramp 157. For instance, the side port 158 may be located a distance proximal of the ramp 157. In some embodiments, the side port 158 may be located about 2 to about 10 centimeters, about 4 to about 6 centimeters, or about 5 centimeters proximal of the ramp 157. Although some suitable dimensions are disclosed, one of skill in the art, incited by the present disclosure, would understand that desired dimensions may deviate from those expressly disclosed. Placing the side port 158 at a location proximal of the ramp 157 may reduce the potential of kinking of the elongate shaft 156.

As shown in FIG. 6A, the ramp 157 may be positioned across a portion of the lumen 163 of the distal section 153. Furthermore, the proximal section 151 may be positioned within the lumen 163 of the distal section 153 such that the perimeter (e.g., circumference) of the first lumen 161 is tangent to the perimeter (e.g., circumference) of the lumen 163 of the distal section 153. Additionally or alternatively, the proximal section 151 may be positioned within the lumen 163 of the distal section 153 such that the perimeter (e.g., circumference) of the second lumen 162 is tangent to the perimeter (e.g., circumference) of the lumen 163 of the distal section 153. As used herein, the term "tangent" is intended to mean a line, curve, or surface meeting another line, curve, or surface at a common point and/or sharing a common tangent line or tangent plane at that point. Thus, as shown in FIG. 6A, the curvature of the circumference of the inner surface of the lumen 163 meets the curvature of the circumference of the inner surface of the first lumen 161 and/or the curvature of the circumference of the inner surface of the second lumen 162, and the lumen 163 shares a common tangent line or tangent plane at that point with the first lumen 161 and/or the second lumen 162. Such an orientation may be found to facilitate advancing an elongate shaft, such as a guidewire, from the lumen 163 of the distal section 153 into the first lumen 161 and/or the second lumen 162 of the proximal section 151.

As shown in FIG. 6B, the first lumen 161, as well as the second lumen 162 may be offset from the longitudinal axis of the proximal section 151. The offset first lumen 161 may facilitate advancing a guidewire through the first lumen 161 and/or through the side port 158. Also in FIG. 6B, the ramp 170 is shown occluding the first lumen 161 of the proximal section 151.

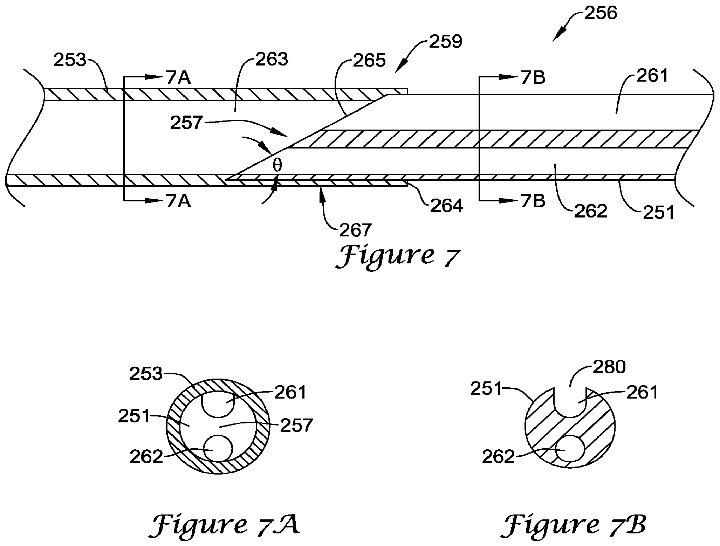

Another alternative embodiment of a portion of an elongate shaft 256 including a joint 259 between a distal section 253 and a proximal section 251 is shown in FIG. 7. In many respects, the portion of the elongate shaft 256 may be similar to the portion of the elongate shaft 56 of the push catheter 50 as shown in FIG. 5. For example, the proximal section 251 may have different flexibility characteristics from the distal section 253. Thus, for the sake of repetitiveness, similarities of the portion of the elongate shaft 256 shown in FIG. 7 with those of the portion of the elongate shaft 56 shown in FIG. 5 will not be repeated.

As shown in FIG. 7B, the proximal section 251 of the elongate shaft 256 shown in FIG. 7 may include a "U" or "C" channel 261 forming a lumen for receiving a guidewire therethrough and a second lumen 262. A longitudinal slot 280 extending along the length, or a portion thereof, of the proximal section 251 may allow access through the sidewall of the proximal section 251 to the "U" or "C" channel 261 of the proximal section 251.

The distal section 253 may be a tubular member having a lumen 263 extending therethrough. Each of the "U" or "C" channel 261 and the lumen 262 of the proximal section 251 may be in association (e.g., in fluid communication) with the lumen 263 of the distal section 253. The "U" or "C" channel 261 allows a guidewire to be selectively retained within the "U" or "C" channel 261, or the guidewire may be removed from the "U" or "C" channel 261, as desired during a medical procedure. Thus, a push catheter including a "U" or "C" channel 261 may be selectively used in an over-the-wire manner and/or in a rapid-exchange manner.

In some embodiments, such as shown in FIG. 7, the distal end 265 of the proximal section 251 may be formed at an oblique angle θ to the longitudinal axis of the proximal section 251. For instance, the oblique angle θ may be in the range of about 10 degrees to about 60 degrees, in the range of about 20 degrees to about 50 degrees, or in the range of about 30 degrees to about 45 degrees in some embodiments. Although some suitable values are disclosed, one of skill in the art, incited by the present disclosure, would understand that desired values may deviate from those expressly disclosed.

The oblique angle θ of the distal end 265 of the proximal section 251 forms a ramp 257 which may be used to facilitate directing a guidewire from the single lumen 263 of the distal section 253 to the "C" or "U" channel 261 of the proximal section 251. The ramp 257 may also be viewed through the lumen 263 of the distal section 253 in the cross-section shown in FIG. 7A.

As shown in the embodiment illustrated in FIG. 7, the distal end 265 of the proximal section 251 may be positioned within the lumen 263 of the distal section 253 and the outer surface of a distal portion of the proximal section 251 secured to the distal section 253. Thus, the outer diameter of the proximal section 251 may be sized to fit within the lumen 263 of the distal section 253. In some embodiments, the lumen 263 of the distal section 253 may have a stepped portion 267 proximate the proximal end 264 of the distal section 253 for receiving the distal portion of the proximal section 251. The internal stepped portion 267 may be bored out, or otherwise have an increased inner diameter relative to a more distal portion of the distal section 253. Thus, in such embodiments, the outer diameter of the proximal section 251 may be similar to the inner diameter of the internal stepped portion 267, and thus may be greater than the inner diameter of a more distal portion of the distal section 253.

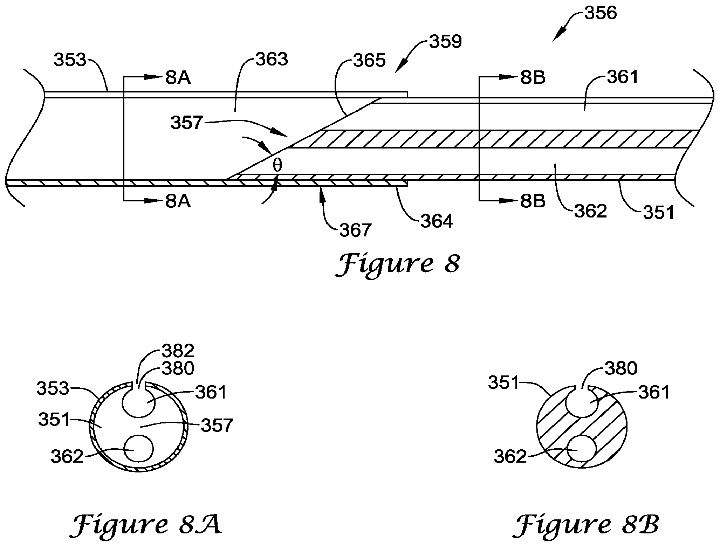

Another alternative embodiment of a portion of an elongate shaft 356 including a joint 359 between a distal section 353 and a proximal section 351 is shown in FIG. 8. In many respects, the portion of the elongate shaft 356 may be similar to the portion of the elongate shaft 56 of the push catheter 50 as shown in FIG. 5. For example, the proximal section 351 may have different flexibility characteristics from the distal section 353. Thus, for the sake of repetitiveness, similarities of the portion of the elongate shaft 356 shown in FIG. 8 with those of the portion of the elongate shaft 56 shown in FIG. 5 will not be repeated.

As shown in FIG. 8A, the distal section 353 of the elongate shaft 356 shown in FIG. 8 may include a tubular member including a longitudinal slot 382 extending along the length, or a portion thereof, of the distal section 353 which may allow access through the sidewall of the distal section 353 to the lumen 363 of the distal section 353.

As shown in FIG. 8B, the proximal section 351 of the elongate shaft 356 shown in FIG. 8 may include a "U" or "C" channel 361 forming a lumen for receiving a guidewire therethrough and a second lumen 362. A longitudinal slot 380 extending along the length, or a portion thereof, of the proximal section 351 may allow access through the sidewall of the proximal section 351 to the "U" or "C" channel 361 of the proximal section 351. As shown in FIG. 8A, the longitudinal slot 380 may be aligned with the longitudinal slot 382.

Each of the "U" or "C" channel 361 and the lumen 362 may be in association (e.g., in fluid communication) with the lumen 363 of the distal section 353. The longitudinal slot 382 of the distal section 353 allows a guidewire to be selectively removed from the confines of the lumen 363 of the distal section 353 and/or inserted into the lumen 363 of the distal section 353 as desired. Additionally and/or alternatively, the "U" or "C" channel 361 of the proximal section 351 allows a guidewire to be selectively retained within the "U" or "C" channel 361, or the guidewire may be removed from the "U" or "C" channel 361 and/or the lumen 363 of the distal section 353, as desired during a medical procedure. Thus, a push catheter including a longitudinal slot 380/382 and/or a "U" or "C" channel 361 may be selectively used in an over-the-wire manner and/or in a rapid-exchange manner.

In some embodiments, such as shown in FIG. 8, the distal end 365 of the proximal section 351 may be formed at an oblique angle θ to the longitudinal axis of the proximal section 351. For instance, the oblique angle θ may be in the range of about 10 degrees to about 60 degrees, in the range of about 20 degrees to about 50 degrees, or in the range of about 30 degrees to about 45 degrees in some embodiments. Although some suitable values are disclosed, one of skill in the art, incited by the present disclosure, would understand that desired values may deviate from those expressly disclosed.

The oblique angle θ of the distal end 365 of the proximal section 351 forms a ramp 357 which may be used to facilitate directing a guidewire from the lumen 363 of the distal section 353 to the "C" or "U" channel 361 of the proximal section 351. The ramp 357 may also be viewed through the lumen 363 of the distal section 353 in the cross-section shown in FIG. 8A.

As shown in the embodiment illustrated in FIG. 8, the distal end 365 of the proximal section 351 may be positioned within the lumen 363 of the distal section 353 and the outer surface of a distal portion of the proximal section 351 secured to the distal section 353. Thus, the outer diameter of the proximal section 351 may be sized to fit within the lumen 363 of the distal section 353. In some embodiments, the lumen 363 of the distal section 353 may have an inner diameter proximate the proximal end 364 of the distal section 353 sized for receiving the distal portion of the proximal section 351. In some embodiments, the outer diameter of the distal portion of the proximal section 351 proximate the distal end 365 of the proximal section 351 may be substantially equal to the inner diameter of the distal section 353 proximate the proximal end 364 of the distal section 353.

FIGS. 9A-9D are a sequence of figures illustrating the advancement of a guidewire 90 through the elongate shaft 56 of the push catheter 50 while retaining the elongate shaft 56 in a curved orientation with the side port 58 positioned at the outer radius of the curved portion of the elongate shaft 56. As shown in the Figures, the pull wire 34 is positioned within the second lumen 62 of the proximal section 51 and the lumen 63 of the distal section 53. The pull wire 34 is positioned, as a result of the curved orientation of the elongate shaft 56, along the inner radius of the curved portion of the elongate shaft 56.

In FIG. 9A, the guidewire 90 is approaching the ramp 57 formed by the oblique angle θ of the distal end 65 of the proximal section 51, yet fully within the distal section 53. The position of the pull wire 34 partially occludes the lumen 63 of the distal section 53, such that the guidewire 90 is located in the radially outward portion of the curve of the lumen 63. As the second lumen 62 is substantially occluded by the pull wire 34, the guidewire 90 is precluded from entering the second lumen 62. As shown in FIG. 9A, due to the curvature of the elongate shaft 56, the leading edge 92 of the guidewire 90 contacts the inner surface of the lumen 63 along the outer radius of the curve. As the guidewire 90 approaches the ramp 57 of the proximal section 51, the ramp 57 facilitates advancing the guidewire 90 into the first lumen 61 of the proximal section 51.

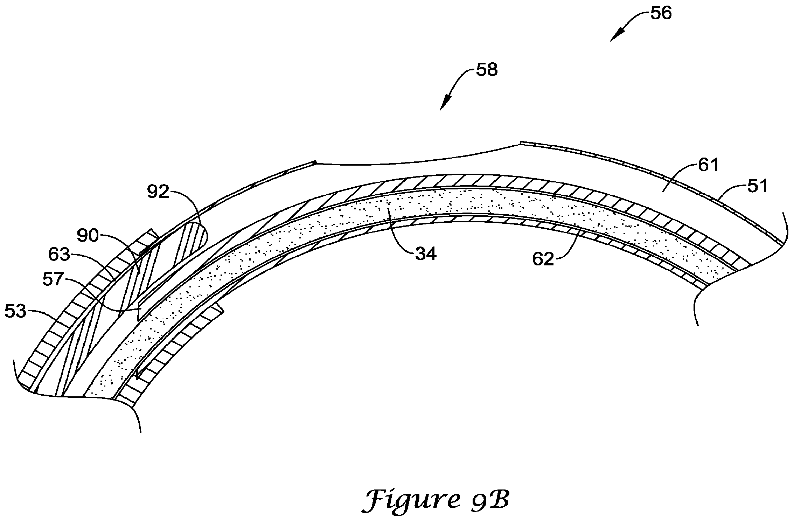

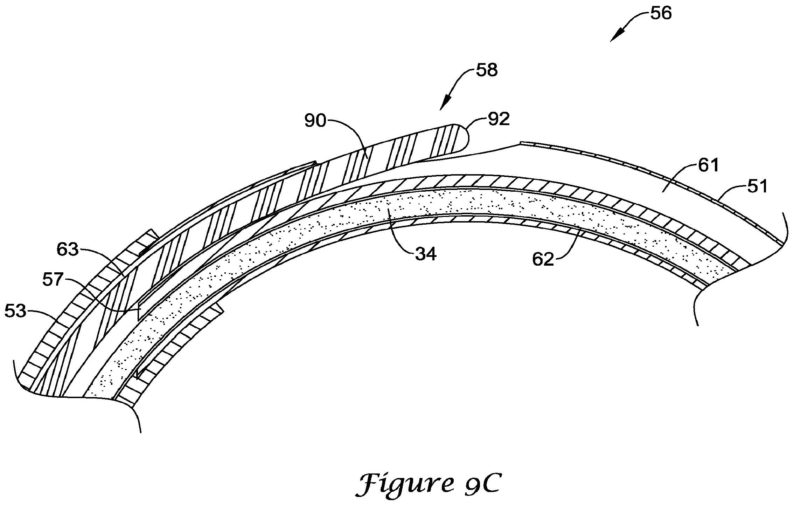

As shown in FIG. 9B, once advanced proximally of the ramp 57, the leading edge 92 of the guidewire 90 further maintains contact with the inner surface of the first lumen 61 along the outer radius of the curve. As the natural tendency of the guidewire 90 is to advance in a straight path, the inner surface of the first lumen 61 along the outer radius of the curve alters the natural tendency of the guidewire 90 and urges the guidewire 90 to follow the curvature of the first lumen 61. As the guidewire 90 is further advanced proximally within the first lumen 61, the leading edge 92 of the guidewire 90 encounters the side port 58, also located along the outer radius of the curve of the elongate shaft 56. Once the leading edge 92 of the guidewire 90 reaches the side port 58, the guidewire 90 exits the lumen 61 through the side port 58, as shown in FIG. 9C. This is due to the fact that through the region of the side port 58, the inner wall of the first lumen 61 no longer impedes the guidewire 90 from advancing in a straight path.

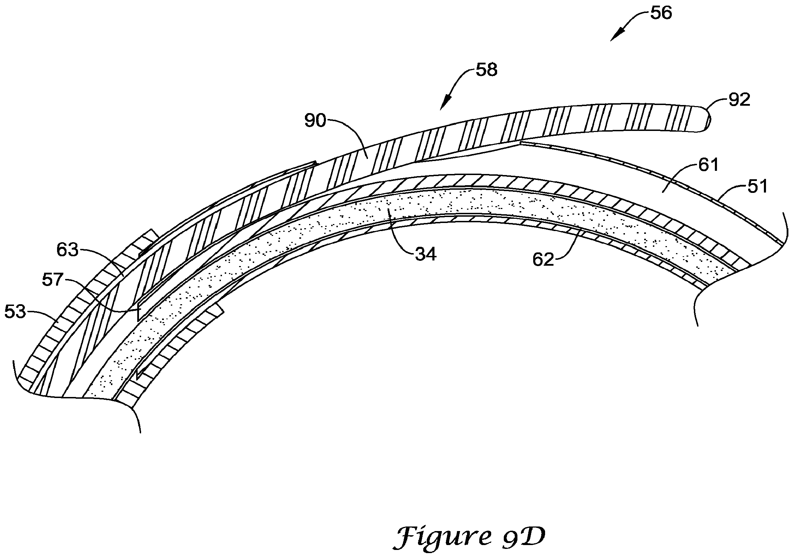

Once the guidewire 90 exits the elongate shaft 56 through the side port 58, the guidewire 90 may be further advanced proximally exterior of the elongate shaft 56, as shown in FIG. 9D. When positioned in this arrangement, the push catheter 50 and guidewire 90 are arranged such that the push catheter 50 may be used as a rapid-exchange catheter.

In some embodiments, where the push catheter 50 is used as a rapid-exchange catheter, a solid mono-filament wire used as the pull wire 34, having a cross-section substantially occluding the second lumen 62, may provide guidewire-like stiffness to the proximal portion of the elongate shaft 56 where the guidewire 90 is positioned external of the elongate shaft 56. Thus, the pull wire 34 may provide attributes associated with an over-the-wire catheter configuration (wherein the guidewire would provide stiffness and pushability to the proximal portion), yet retain the benefits of a rapid-exchange catheter configuration.

In procedures wherein the push catheter 50 is desired to be used as an over-the-wire catheter, the portion of the elongate shaft 56 including the side port 58 may be retained in a straight configuration during advancement of the guidewire 90 through the elongate shaft 56. Thus, during advancement of the guidewire 90, instead of exiting the elongate shaft 56 through the side port 58, the guidewire passes the side port 58 of the proximal section 51 and advances through the first lumen 61 proximally of the side port 58. This is due to the fact that as the natural tendency of the guidewire 90 is to advance in a straight path, the guidewire 90 will remain in the straight path created by the first lumen 61.

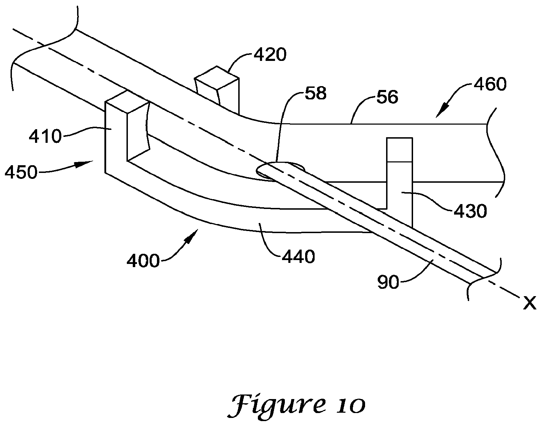

A tool 400 which may be used to urge the elongate shaft 56 of the push catheter 50 into a curved orientation is shown in FIG. 10. The tool 400 includes a body portion 440, a securement portion 450 including a first leg 410 and a second leg 420, and biasing portion 460 including a third leg 430. Each of the first, second and third legs 410, 420, 430 extend from the body portion 440. The first leg 410 and the second leg 420 are arranged such that the first and second legs 410, 420 bound the elongate shaft 56 on opposing sides. The first leg 410 and the second leg 420 may be opposite or slightly offset from one another. The first and second legs 410, 420 may include a curved portion such as a concave surface, a protrusion, or otherwise may include structure for retaining the elongate shaft 56 between the first and second legs 410, 420. Thus, in some embodiments, the first and second legs 410, 420 may provide an interference or interlocking fit with the elongate shaft 56.

The third leg 430 may be placed a distance from the first and second legs 410, 420. The third leg 430 may be misaligned from the first and second legs 410, 420 such that placement of the elongate shaft 56 adjacent to the third leg 430 biases the elongate shaft 56, imparting a curvature in the elongate shaft 56. The third leg 430 may additionally or alternatively include a curved portion such as a concave surface, a protrusion, or otherwise may include structure for retaining the elongate shaft 56.

The elongate shaft 56 may be positioned such that the side port 58 is positioned between the securement portion 450 (i.e., the first and second legs 410, 420) and the biasing portion 460 (i.e., the third leg 430) of the tool 400. Thus, the side port 58 may be positioned along the outer radius of the curvature of the elongate shaft 56 formed between the first and second legs 410, 420 and the third leg 430 in an orientation similar to that illustrated in FIGS. 9A-9D. Thus, as the guidewire 90 is advanced through the elongate shaft 56, the tool 400, which provides a curvature to the elongate shaft 56, may facilitate the guidewire 90 exiting the elongate shaft 56 through the side port 58 such as illustrated in FIGS. 9A-9D.

Those skilled in the art will recognize that the present invention may be manifested in a variety of forms other than the specific embodiments described and contemplated herein. Accordingly, departure in form and detail may be made without departing from the scope of the present invention as described in the appended claims.

## Claims

1. A catheter shaft for use in a medical procedure, the catheter shaft comprising:
a distal segment (53; 153; 253; 353) having a proximal end, a distal end, an internal stepped portion (67; 267) adjacent the proximal end and having an increased inner diameter relative to a more distant portion of the distal segment (53; 153; 253; 353), and a lumen (63; 163; 263) extending therethrough; and
a multi-lumen proximal segment (51; 151; 251; 351) having a proximal end, a distal end, a first lumen (61; 161; 261) and a second lumen (62; 162; 262), wherein the distal end of the proximal segment (51; 151; 251; 351) is formed at an oblique angle defining a ramp (57; 157; 257);
wherein the distal segment (53; 153; 253; 353) is secured to the proximal segment (51; 151; 251; 351) such that the distal end of the proximal segment (51) is positioned within the lumen (63) of the distal segment, and that each of the first and second lumens of the proximal segment (51; 151; 251; 351) is in association with the lumen (63; 163; 263) of the distal segment (53; 153; 253; 353).

2. The catheter shaft of claim 1, wherein the proximal segment (51; 151; 251; 351) further includes a side port (58) providing access to the first lumen (61; 161; 261) of the proximal segment (51; 151; 251; 351).

3. The catheter shaft of claim 2, wherein the side port (58) is located about 2 to about 10 centimeters proximal of the ramp (57; 157; 257).

4. The catheter shaft of claim 2, wherein the side port (58) is located about 4 to about 6 centimeters proximal of the ramp (57; 157; 257).

5. The catheter shaft of claim 1, wherein the proximal segment (51; 151; 251; 351) has an outer diameter and the distal segment (53; 153; 253; 353) has an outer diameter greater than the outer diameter of the proximal segment (51; 151; 251; 351).

6. The catheter shaft of claim 1, wherein the proximal segment (51; 151; 251; 351) has a durometer hardness and the distal segment (53; 153; 253; 353) has a durometer hardness different from the durometer hardness of the proximal segment (51; 151; 251; 351).

7. The catheter shaft of claim 6, wherein the proximal segment (51; 151; 251; 351) has a durometer hardness in the range of about 70D to about 80D on the Shore hardness scale.

8. The catheter shaft of claim 6, wherein the distal segment (53; 153; 253; 353) has a durometer hardness in the range of about 50D to about 70D on the Shore hardness scale.

9. The catheter shaft of claim 6, wherein the proximal segment (51; 151; 251; 351) is formed of a first polymer and the distal segment (53; 153; 253; 353) is formed of a second polymer different from the first polymer.

10. The catheter shaft of claim 1, wherein the first lumen (61; 161; 261) of the proximal segment (51; 151; 251; 351) has an inner surface and the lumen of the distal segment (53; 153; 253; 353) has an inner surface,
wherein the inner surface of the first lumen (61; 161; 261) of the proximal segment (51; 151; 251; 351) is tangent to the inner surface of the lumen (63; 163; 263) of the distal segment (53; 153; 253; 353).

11. The catheter shaft of claim 1, wherein the proximal segment (51; 151; 251; 351) includes a longitudinal slot (280; 380) providing access to the first lumen (61; 161; 261) of the proximal segment (51; 151; 251; 351).

12. The catheter shaft of claim 11, wherein the distal segment (53; 153; 253; 353) includes a longitudinal slot (382) providing access to the lumen (63; 163; 263) of the distal segment (53; 153; 253; 353).

13. The catheter shaft of claim 12, wherein the longitudinal slot (280; 380) of the proximal segment (51; 151; 251; 351) is aligned with the longitudinal slot (382) of the distal segment (53; 153; 253; 353).

14. The catheter shaft of claim 1, further comprising:
a hub assembly (54) secured to the proximal end of the proximal segment (51; 151; 251; 351); and
a side port (58) extending through a side wall of the proximal segment (51; 151; 251; 351) and providing access to the first lumen.

15. The catheter shaft of claim 14, wherein the distal end of the proximal segment (51; 151; 251; 351) of the catheter shaft is formed at an angle of about 20 degrees to about 50 degrees.

16. The catheter shaft of claim 14, wherein the distal end of the proximal segment (51; 151; 251; 351) of the catheter shaft is formed at an angle of about 30 degrees to about 45 degrees.

17. The catheter shaft of claim 14, wherein the side port (58) is located about 2 to about 10 centimeters proximal of the ramp (57; 157; 257).

18. The catheter shaft of claim 14, wherein the side port (58) is located about 4 to about 6 centimeters proximal of the ramp (57; 157; 257).

19. The catheter shaft of claim 14, wherein the distal segment (53; 153; 253; 353) has a first inner diameter, wherein the stepped portion (67; 267) of the distal segment (53; 153; 253; 353) has a second inner diameter greater than the first inner diameter.

20. The catheter shaft of claim 19, wherein the proximal segment (51; 151; 251; 351) has an outer diameter greater than the first inner diameter of the distal segment (53; 153; 253; 353), such that a distal portion of the proximal segment (51; 151; 251; 351) is positioned within the stepped portion (67; 267) of the distal segment (53; 153; 253; 353).

21. The catheter shaft of claim 14, wherein the first lumen (61; 161; 261) of the proximal segment (51; 151; 251; 351) has an inner surface and the lumen (63; 163; 263) of the distal segment (53; 153; 253; 353) has an inner surface,
wherein the inner surface of the first lumen (61; 161; 261) of the proximal segment (51; 151; 251; 351) is tangent to the inner surface of the lumen (63; 163; 263) of the distal segment (53; 153; 253; 353).

22. The catheter shaft of claim 14, wherein the proximal segment (51; 151; 251; 351) includes a longitudinal slot (280; 380) providing access to the first lumen (61; 161; 261) of the proximal segment (51; 151; 251; 351).

23. The catheter shaft of claim 22, wherein the distal segment (53; 153; 253; 353) includes a longitudinal slot (382) providing access to the lumen (63; 163; 263) of the distal segment (53; 153; 253; 353).

24. The catheter shaft of claim 23, wherein the longitudinal slot (280; 380) of the proximal segment (51; 151; 251; 351) is aligned with the longitudinal slot (382) of the distal segment (53; 153; 253; 353).

## Patentansprüche

1. Katheterschaft zur Verwendung in einer medizinischen Prozedur, wobei der Katheterschaft aufweist:
ein distales Segment (53; 153; 253; 353) mit einem proximalen Ende, einem distalen Ende, einem abgestuften Innenabschnitt (67; 267), der benachbart zum proximalen Ende ist und einen vergrößerten Innendurchmesser relativ zu einem entfernteren Abschnitt des distalen Segments (53; 153; 253; 353) hat, und einem Lumen (63; 163; 263), das sich dadurch erstreckt; und
ein proximales Mehrlumensegment (51; 151; 251; 351) mit einem proximalen Ende, einem distalen Ende, einem ersten Lumen (61; 161; 261) und einem zweiten Lumen (62; 162; 262), wobei das distale Ende des proximalen Segments (51; 151; 251; 351) in einem schrägen Winkel ausgebildet ist, der eine Rampe (57; 157; 257) bildet;
wobei das distale Segment (53; 153; 253; 353) am proximalen Segment (51; 151; 251; 351) so befestigt ist, dass das distale Ende des proximalen Segments (51) im Lumen (63) des distalen Segments positioniert ist und dass das erste und zweite Lumen des proximalen Segments (51; 151; 251; 351) jeweils dem Lumen (63; 163; 263) des distalen Segments (53; 153; 253; 353) zugeordnet sind.

2. Katheterschaft nach Anspruch 1, wobei das proximale Segment (51; 151; 251; 351) ferner einen Seiten-Port (58) aufweist, der für Zugang zum ersten Lumen (61; 161; 261) des proximalen Segments (51; 151; 251; 351) sorgt.

3. Katheterschaft nach Anspruch 2, wobei der Seiten-Port (58) etwa 2 bis etwa 10 Zentimeter proximal von der Rampe (57; 157; 257) liegt.

4. Katheterschaft nach Anspruch 2, wobei der Seiten-Port (58) etwa 4 bis etwa 6 Zentimeter proximal von der Rampe (57; 157; 257) liegt.

5. Katheterschaft nach Anspruch 1, wobei das proximale Segment (51; 151; 251; 351) einen Außendurchmesser hat und das distale Segment (53; 153; 253; 353) einen Außendurchmesser hat, der größer als der Außendurchmesser des proximalen Segments (51; 151; 251; 351) ist.

6. Katheterschaft nach Anspruch 1, wobei das proximale Segment (51; 151; 251; 351) eine Durometerhärte hat und das distale Segment (53; 153; 253; 353) eine Durometerhärte hat, die sich von der Durometerhärte des proximalen Segments (51; 151; 251; 351) unterscheidet.

7. Katheterschaft nach Anspruch 6, wobei das proximale Segment (51; 151; 251; 351) eine Durometerhärte im Bereich von etwa 70D bis etwa 80D auf der Shore-Härteskala hat.

8. Katheterschaft nach Anspruch 6, wobei das distale Segment (53; 153; 253; 353) eine Durometerhärte im Bereich von etwa 50D bis etwa 70D auf der Shore-Härteskala hat.

9. Katheterschaft nach Anspruch 6, wobei das proximale Segment (51; 151; 251; 351) aus einem ersten Polymer gebildet ist und das distale Segment (53; 153; 253; 353) aus einem zweiten Polymer gebildet ist, das sich vom ersten Polymer unterscheidet.

10. Katheterschaft nach Anspruch 1, wobei das erste Lumen (61; 161; 261) des proximalen Segments (51; 151; 251; 351) eine Innenfläche hat und das Lumen des distalen Segments (53; 153; 253; 353) eine Innenfläche hat,
wobei die Innenfläche des ersten Lumens (61; 161; 261) des proximalen Segments (51; 151; 251; 351) tangential zur Innenfläche des Lumens (63; 163; 263) des distalen Segments (53; 153; 253; 353) ist.

11. Katheterschaft nach Anspruch 1, wobei das proximale Segment (51; 151; 251; 351) einen Längsschlitz (280; 380) aufweist, der für Zugang zum ersten Lumen (61; 161; 261) des proximalen Segments (51; 151; 251; 351) sorgt.

12. Katheterschaft nach Anspruch 11, wobei das distale Segment (53; 153; 253; 353) einen Längsschlitz (382) aufweist, der für Zugang zum Lumen (63; 163; 263) des distalen Segments (53; 153; 253; 353) sorgt.

13. Katheterschaft nach Anspruch 12, wobei der Längsschlitz (280; 380) des proximalen Segments (51; 151; 251; 351) zum Längsschlitz (382) des distalen Segments (53; 153; 253; 353) ausgerichtet ist.

14. Katheterschaft nach Anspruch 1, der ferner aufweist:
eine Hub-Anordnung (54), die am proximalen Ende des proximalen Segments (51; 151; 251; 351) befestigt ist; und
einen Seiten-Port (58), der sich durch eine Seitenwand des proximalen Segments (51; 151; 251; 351) erstreckt und für Zugang zum ersten Lumen sorgt.

15. Katheterschaft nach Anspruch 14, wobei das distale Ende des proximalen Segments (51; 151; 251; 351) des Katheterschafts in einem Winkel von etwa 20 Grad bis etwa 50 Grad ausgebildet ist.

16. Katheterschaft nach Anspruch 14, wobei das distale Ende des proximalen Segments (51; 151; 251; 351) des Katheterschafts in einem Winkel von etwa 30 Grad bis etwa 45 Grad ausgebildet ist.

17. Katheterschaft nach Anspruch 14, wobei der Seiten-Port (58) etwa 2 bis etwa 10 Zentimeter proximal von der Rampe (57; 157; 257) liegt.

18. Katheterschaft nach Anspruch 14, wobei der Seiten-Port (58) etwa 4 bis etwa 6 Zentimeter proximal von der Rampe (57; 157; 257) liegt.

19. Katheterschaft nach Anspruch 14, wobei das distale Segment (53; 153; 253; 353) einen ersten Innendurchmesser hat, wobei der abgestufte Abschnitt (67; 267) des distalen Segments (53; 153; 253; 353) einen zweiten Innendurchmesser hat, der größer als der erste Innendurchmesser ist.

20. Katheterschaft nach Anspruch 19, wobei das proximale Segment (51; 151; 251; 351) einen Außendurchmesser hat, der größer als der erste Innendurchmesser des distalen Segments (53; 153; 253; 353) ist, so dass ein distaler Abschnitt des proximalen Segments (51; 151; 251; 351) im abgestuften Abschnitt (67; 267) des distalen Segments (53; 153; 253; 353) positioniert ist.

21. Katheterschaft nach Anspruch 14, wobei das erste Lumen (61; 161; 261) des proximalen Segments (51; 151; 251; 351) eine Innenfläche hat und das Lumen (63; 163; 263) des distalen Segments (53; 153; 253; 353) eine Innenfläche hat,
wobei die Innenfläche des ersten Lumens (61; 161; 261) des proximalen Segments (51; 151; 251; 351) tangential zur Innenfläche des Lumens (63; 163; 263) des distalen Segments (53; 153; 253; 353) ist.

22. Katheterschaft nach Anspruch 14, wobei das proximale Segment (51; 151; 251; 351) einen Längsschlitz (280; 380) aufweist, der für Zugang zum ersten Lumen (61; 161; 261) des proximalen Segments (51; 151; 251; 351) sorgt.

23. Katheterschaft nach Anspruch 22, wobei das distale Segment (53; 153; 253; 353) einen Längsschlitz (382) aufweist, der für Zugang zum Lumen (63; 163; 263) des distalen Segments (53; 153; 253; 353) sorgt.

24. Katheterschaft nach Anspruch 23, wobei der Längsschlitz (280; 380) des proximalen Segments (51; 151; 251; 351) zum Längsschlitz (382) des distalen Segments (53; 153; 253; 353) ausgerichtet ist.

## Revendications

1. Tige de cathéter destinée à être utilisée dans une opération médicale, la tige de cathéter comprenant :
un segment distal (53 ; 153 ; 253 ; 353) ayant une extrémité proximale, une extrémité distale, une partie étagée interne (67 ; 267) adjacente à l'extrémité proximale et ayant un diamètre interne augmenté par rapport à une partie plus distante du segment distal (53 ; 153 ; 253 ; 353), et une lumière (63 ; 163 ; 263) qui s'étend à travers celui-ci ; et
un segment proximal à lumières multiples (51 ; 151 ; 251 ; 351) ayant une extrémité proximale, une extrémité distale, une première lumière (61 ; 161 ; 261) et une seconde lumière (62 ; 162 ; 262), où l'extrémité distale du segment proximal (51 ; 151 ; 251 ; 351) est formée sous un angle oblique définissant une rampe (57 ; 157 ; 257) ;
où le segment distal (53 ; 153 ; 253 ; 353) est fixé au segment proximal (51 ; 151 ; 251 ; 351) de sorte que l'extrémité distale du segment proximal (51) est positionnée dans la lumière (63) du segment distal, et que chacune de la première et de la seconde lumière du segment proximal (51 ; 151 ; 251 ; 351) est en association avec la lumière (63 ; 163 ; 263) du segment distal (53 ; 153 ; 253 ; 353).

2. Tige de cathéter selon la revendication 1, où le segment proximal (51 ; 151 ; 251 ; 351) inclut en outre un orifice latéral (58) permettant un accès à la première lumière (61 ; 161 ; 261) du segment proximal (51 ; 151 ; 251 ; 351).

3. Tige de cathéter selon la revendication 2, où l'orifice latéral (58) est situé à environ 2 à environ 10 centimètres de manière proximale par rapport à la rampe (57 ; 157 ; 257).

4. Tige de cathéter selon la revendication 2, où l'orifice latéral (58) est situé à environ 4 à environ 6 centimètres de manière proximale par rapport à la rampe (57 ; 157 ; 257).

5. Tige de cathéter selon la revendication 1, où le segment proximal (51 ; 151 ; 251 ; 351) a un diamètre externe et le segment distal (53 ; 153 ; 253 ; 353) a un diamètre externe plus grand que le diamètre externe du segment proximal (51 ; 151 ; 251 ; 351).

6. Tige de cathéter selon la revendication 1, où le segment proximal (51 ; 151 ; 251 ; 351) a une dureté au duromètre et le segment distal (53 ; 153 ; 253 ; 353) a une dureté au duromètre différente de la dureté au duromètre du segment proximal (51 ; 151 ; 251 ; 351).

7. Tige de cathéter selon la revendication 6, où le segment proximal (51 ; 151 ; 251 ; 351) a une dureté au duromètre dans la plage d'environ 70D à environ 80D sur l'échelle de dureté Shore.

8. Tige de cathéter selon la revendication 6, où le segment distal (53 ; 153 ; 253 ; 353) a une dureté au duromètre dans la plage d'environ 50D à environ 70D sur l'échelle de dureté Shore.

9. Tige de cathéter selon la revendication 6, où le segment proximal (51 ; 151 ; 251 ; 351) est formé d'un premier polymère et le segment distal (53 ; 153 ; 253 ; 353) est formé d'un second polymère différent du premier polymère.

10. Tige de cathéter selon la revendication 1, où la première lumière (61 ; 161 ; 261) du segment proximal (51 ; 151 ; 251 ; 351) a une surface interne et la lumière du segment distal (53 ; 153 ; 253 ; 353) a une surface interne,
où la surface interne de la première lumière (61 ; 161 ; 261) du segment proximal (51 ; 151 ; 251 ; 351) est tangente à la surface interne de la lumière (63 ; 163 ; 263) du segment distal (53 ; 153 ; 253 ; 353).

11. Tige de cathéter selon la revendication 1, où le segment proximal (51 ; 151 ; 251 ; 351) inclut une fente longitudinale (280 ; 380) permettant un accès à la première lumière (61 ; 161 ; 261) du segment proximal (51 ; 151 ; 251 ; 351).

12. Tige de cathéter selon la revendication 11, où le segment distal (53 ; 153 ; 253 ; 353) inclut une fente longitudinale (382) permettant un accès à la lumière (63 ; 163 ; 263) du segment distal (53 ; 153 ; 253 ; 353).

13. Tige de cathéter selon la revendication 12, où la fente longitudinale (280 ; 380) du segment proximal (51 ; 151 ; 251 ; 351) est alignée avec la fente longitudinale (382) du segment distal (53 ; 153 ; 253 ; 353).

14. Tige de cathéter selon la revendication 1, comprenant en outre :
un ensemble formant moyeu (54) fixé à l'extrémité proximale du segment proximal (51 ; 151 ; 251 ; 351) ; et
un orifice latéral (58) qui s'étend à travers une paroi latérale du segment proximal (51 ; 151 ; 251 ; 351) et permettant un accès à la première lumière.

15. Tige de cathéter selon la revendication 14, où l'extrémité distale du segment proximal (51 ; 151 ; 251 ; 351) de la tige de cathéter est formée sous un angle d'environ 20 degrés à environ 50 degrés.

16. Tige de cathéter selon la revendication 14, où l'extrémité distale du segment proximal (51 ; 151 ; 251 ; 351) de la tige de cathéter est formée sous un angle d'environ 30 degrés à environ 45 degrés.

17. Tige de cathéter selon la revendication 14, où l'orifice latéral (58) est situé à environ 2 à environ 10 centimètres de manière proximale par rapport à la rampe (57 ; 157 ; 257).

18. Tige de cathéter selon la revendication 14, où l'orifice latéral (58) est situé à environ 4 à environ 6 centimètres de manière proximale par rapport à la rampe (57 ; 157 ; 257).

19. Tige de cathéter selon la revendication 14, où le segment distal (53 ; 153 ; 253 ; 353) a un premier diamètre interne, où la partie étagée (67 ; 267) du segment distal (53 ; 153 ; 253 ; 353) a un second diamètre interne plus grand que le premier diamètre interne.

20. Tige de cathéter selon la revendication 19, où le segment proximal (51 ; 151 ; 251 ; 351) a un diamètre externe plus grand que le premier diamètre interne du segment distal (53 ; 153 ; 253 ; 353), de sorte qu'une partie distale du segment proximal (51 ; 151 ; 251 ; 351) est positionnée dans la partie étagée (67 ; 267) du segment distal (53 ; 153 ; 253 ; 353).

21. Tige de cathéter selon la revendication 14, où la première lumière (61 ; 161 ; 261) du segment proximal (51 ; 151 ; 251 ; 351) a une surface interne et la lumière (63 ; 163 ; 263) du segment distal (53 ; 153 ; 253 ; 353) a une surface interne,
où la surface interne de la première lumière (61 ; 161 ; 261) du segment proximal (51 ; 151 ; 251 ; 351) est tangente à la surface interne de la lumière (63 ; 163 ; 263) du segment distal (53 ; 153 ; 253 ; 353).

22. Tige de cathéter selon la revendication 14, où le segment proximal (51 ; 151 ; 251 ; 351) inclut une fente longitudinale (280 ; 380) permettant un accès à la première lumière (61 ; 161 ; 261) du segment proximal (51 ; 151 ; 251 ; 351).

23. Tige de cathéter selon la revendication 22, où le segment distal (53 ; 153 ; 253 ; 353) inclut une fente longitudinale (382) permettant un accès à la lumière (63 ; 163 ; 263) du segment distal (53 ; 153 ; 253 ; 353).

24. Tige de cathéter selon la revendication 23, où la fente longitudinale (280 ; 380) du segment proximal (51 ; 151 ; 251 ; 351) est alignée avec la fente longitudinale (382) du segment distal (53 ; 153 ; 253 ; 353).
